# EUROPEAN PATENT APPLICATION

(11) **EP 3 804 640 A1**
(43) Date of publication of application: **14.04.2021**
(21) Application number: 18919552.2
(22) Date of filing: 24.05.2018
(51) Int. Cl.: A61B 17/34, A61M 25/06

(54) **DILATOR**

(71) Applicant: ASAHI INTECC CO., LTD., Seto-shi, Aichi 489-0071 (JP)
(72) Inventor: FUSEYA, Yukihiro, Seto-shi, Aichi 489-0071 (JP); TSUZUKU, Marina, Seto-shi, Aichi 489-0071 (JP)
(74) Representative: Prinz & Partner mbB
(86) International application number: PCT/JP2018/019981
(87) International publication number: WO 2019/224970

(57) **Abstract**

A dilator capable of easily and securely expanding a hole in an object is provided.

The dilator 1 includes a hollow shaft 11 and a spirally-arranged protruding portion 21. The shaft 11 has a tapered portion 111, a distal end portion 112 positioned on a distal end of the tapered portion 111, and a main body 113 positioned on a proximal end of the tapered portion 111, or alternatively the shaft does not have the distal end portion 112 but has the tapered portion 111 and the main body 113. When the shaft 11 has no distal end portion 112, a height of a spirally-arranged protruding portion 211 formed on the tapered portion 111 is larger than a height of a spirally-arranged protruding portion 213 formed on the main body 113. When the shaft 11 has the distal end portion 112, the height t1 of the spirally-arranged protruding portion 211 on the tapered portion 111 is larger than a height t2 of the spirally-arranged protruding portion 212 on the distal end portion 112 and/or a height t3 of the spirally-arranged protruding portion 213 on the main body 113.

## Description

### TECHNICAL FIELD

The present invention relates to a dilator.

### BACKGROUND ART

As an instrument for expanding a hole formed on a wall of an organ such as stomach and liver, or a constricted part in a body cavity such as bile duct and pancreatic duct, for example, a dilator is known. When using such a dilator, a distal end of the dilator is inserted into the aforementioned hole or the like, and a tapered portion of the dilator is pushed into the hole, so that the hole is expanded (e.g. see Patent Literature 1).

### CITATION LIST

### Patent Literature

Patent Literature 1: Japanese Unexamined Patent Application Publication No. 2008-11867

### SUMMARY OF INVENTION

### TECHNICAL PROBLEM

However, when using the conventional dilator as described above, a sufficient propulsion force cannot be ensured particularly on a tapered portion where an indentation resistance to a hole or a constricted part increases, and there is a concern that the hole and the like cannot be sufficiently expanded.

The present invention has been made on the basis of the aforementioned circumstances, and an object of the present invention is to provide a dilator that can easily and securely expand a hole formed on a wall of an organ, a constricted part, or the like (hereinafter, also referred to as a "hole of object").

### SOLUTION TO PROBLEM

Some aspects of the present disclosure include
(1) a dilator including a hollow shaft and a spirally-arranged protruding portion, in which
   the shaft includes either
   a tapered portion having an outer diameter that is smaller at a distal end of the tapered portion than at a proximal end of the tapered portion,
   a distal end portion having a proximal end positioned on a distal end of the tapered portion and extending toward an axis-directional distal end side of the tapered portion, and
   a main body having a distal end positioned on a proximal end of the tapered portion and extending toward an axis-directional proximal end side of the tapered portion, or
   the tapered portion and the main body without the distal end portion, and
   when the shaft does not have the distal end portion, the spirally-arranged protruding portion is formed on each of outer peripheral surfaces of the tapered portion and the main body and has gaps between adjacent sections along an axial direction of the shaft, and
   the spirally-arranged protruding portion formed on the tapered portion has a height larger than a height of the spirally-arranged protruding portion formed on the main body, and
   when the shaft has the distal end portion, the spirally-arranged protruding portion is formed on each of outer peripheral surfaces of the tapered portion, and the distal end portion and/or the main body, and has gaps between adjacent sections along the axial direction of the shaft, and
   the spirally-arranged protruding portion formed on the tapered portion has a height larger than heights of the spirally-arranged protruding portions formed on the distal end portion and/or the main body,
(2) the dilator according to (1), in which the shaft is composed of a first coil body including a wire wound around an axis of the shaft,
(3) the dilator according to (1) or (2), in which the spirally-arranged protruding portion is composed of a second coil body including a wire wound around on the outer peripheral surface of the shaft,
(4) the dilator according to (1), in which the shaft is composed of the first coil body including a wire wound around an axis of the shaft, and the spirally-arranged protruding portion is composed of the second coil body including a wire wound around on the outer peripheral surface of the shaft, and a winding direction of the wire on the first coil body and a winding direction of the wire on the second coil body are opposite to each other,
(5) the dilator according to (3) or (4), in which the wire of the second coil body wound around the outer peripheral surface of the tapered portion has a diameter larger than a diameter of the wire of the second coil body wound around on the outer peripheral surface of the shaft excluding the tapered portion,
(6) the dilator according to any one of (1) to (5), in which a pitch of the spirally-arranged protruding portion formed on the outer peripheral surface of the tapered portion is smaller than a pitch of the spirally-arranged protruding portion formed on the outer peripheral surface of the shaft excluding the tapered portion, and
(7) a dilator including a hollow shaft and a spirally-arranged protruding portion, in which
   the shaft includes either
   a tapered portion having an outer diameter that is smaller at a distal end of the tapered portion than at a proximal end of the tapered portion,
   a distal end portion having a proximal end positioned on a distal end of the tapered portion and extending toward an axis-directional distal end side of the tapered portion, and
   a main body having a distal end positioned on a proximal end of the tapered portion and extending toward an axis-directional proximal end side of the tapered portion, or
   the tapered portion and the main body without the distal end portion, and
   when the shaft does not have the distal end portion, the spirally-arranged protruding portion is formed on each of outer peripheral surfaces of the tapered portion and the main body, and has gaps between adjacent sections along an axial direction of the shaft, and
   the spirally-arranged protruding portion formed on the tapered portion has a height smaller than a height of the spirally-arranged protruding portion formed on the main body, and
   when the shaft has the distal end portion, the spirally-arranged protruding portion is formed on each of the outer peripheral surfaces of the tapered portion, and the distal end portion and/or the main body, and has gaps between adjacent sections along the axial direction of the shaft, and
   the spirally-arranged protruding portion formed on the tapered portion has a height smaller than heights of the spirally-arranged protruding portions formed on the distal end portion and/or the main body.

Incidentally, the "distal end side" herein means a direction along the axial direction of the shaft in which the tapered portion is positioned with respect to the main body. In addition, the "proximal end side" means a direction along the axial direction of the dilator and opposite to the distal end side. In addition, the "distal end" refers to an end portion of the distal end side on any member or part, and the "proximal end" refers to an end portion of the proximal end side on any member or part. In addition, the "height of the protruding portion" means a size from the outer peripheral surface of the shaft where the spirally-arranged protruding portion is positioned to the outer peripheral end of the spirally-arranged protruding portion when viewed from the front in the axial direction of the shaft, and specifically, for example, means each of heights t1 to t3 of the spirally-arranged protruding portion in each figure herein.

### ADVANTAGEOUS EFFECTS OF INVENTION

The disclosed embodiments can provide a dilator capable of easily and securely expanding a hole in an object.

### BRIEF DESCRIPTION OF DRAWINGS

FIG. 1 is a partially cut-out schematic side view illustrating the whole of the first embodiment of the disclosed embodiments.
FIG. 2 is a schematic enlarged side view of a main part illustrating a variation of FIG. 1.
FIG. 3 is a partially cut-out schematic enlarged side view of a main part illustrating a variation of FIG. 1.
FIG. 4 is a partially cut-out schematic enlarged side view of a main part illustrating a variation of FIG. 1.
FIG. 5 is a partially cut-out schematic enlarged side view of a main part illustrating the second embodiment of the disclosed embodiments.
FIG. 6 is a partially cut-out schematic enlarged side view of a main part illustrating the third embodiment of the disclosed embodiments.
FIG. 7 is a schematic side view illustrating the whole of the fourth embodiment of disclosed embodiments.
FIG. 8 is a schematic enlarged side view of a main part illustrating the fifth embodiment of the disclosed embodiments.
FIG. 9 is a partially cut-out schematic enlarged side view of a main part illustrating the sixth embodiment of the disclosed embodiments.
FIG. 10 is a partially cut-out schematic enlarged side view of a main part illustrating a variation of FIG. 9.
FIG. 11 is a partially cut-out schematic enlarged side view of a main part illustrating a variation of FIG. 9.
FIG. 12 is a partially cut-out schematic enlarged side view of a main part illustrating the seventh embodiment of the disclosed embodiments.

### DESCRIPTION OF EMBODIMENTS

Hereinafter, the first to seventh embodiments of the present invention will be explained with reference to the drawings, but the present invention is not limited to the embodiments illustrated in the FIGs. In addition, sizes of the dilator illustrated in each figure are described to make it easier to understand the contents of the embodiments, and do not correspond to the actual sizes.

### [First Embodiment]

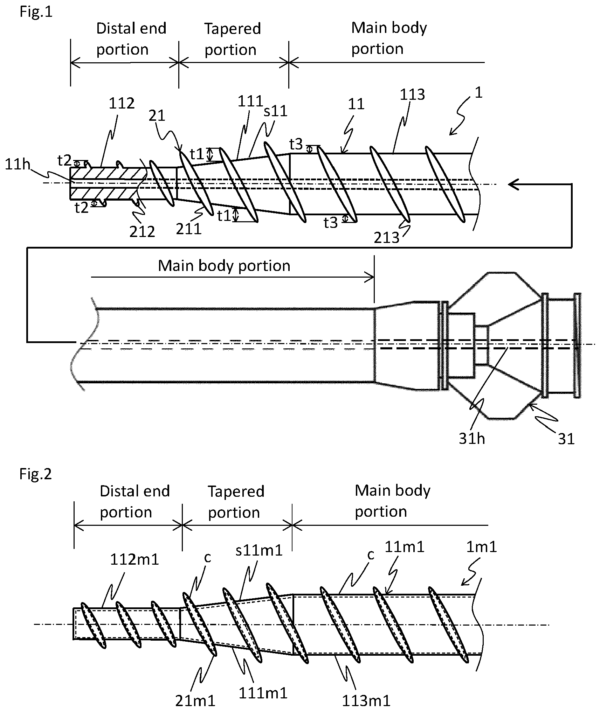

FIG. 1 is a partially cut-out schematic side view illustrating the whole of the first embodiment of the present invention. As illustrated in FIG. 1, a dilator 1 is roughly composed of a shaft 11, a spirally-arranged protruding portion 21, and a base portion 31.

The shaft 11 is a hollow member having a through-hole 11h. The through-hole 11h is e.g. a through-hole through which a guide wire (not illustrated) or the like is passed, and is composed of a continuous space that connects a distal end and a proximal end of the shaft 11 such that the guide wire and the like can freely pass. This shaft 11 has a tapered portion 111, a distal end portion 112, and a main body 113.

The tapered portion 111 is a part whose outer diameter at its distal end is smaller than at its proximal end. Specifically, the tapered portion 111 is connected to a distal end of the main body 113 described below, extends from this distal end toward a distal end side, and has a shape tapered toward the distal end side.

The distal end portion 112 is a part having a proximal end positioned on the distal end of the tapered portion 111 and extending toward the axis-directional distal end side of the tapered portion 111. Specifically, the distal end portion 112 has e.g. a proximal end continuous with the distal end of the tapered portion 111 and has a substantially constant outer diameter from the distal end to the proximal end. Incidentally, the distal end portion 112 can be formed integrally with or separately from the tapered portion 111. In the present embodiment, the distal end portion 112 and the tapered portion 111 are integrally formed by casting or the like.

The main body 113 is a part having the distal end positioned on the proximal end of the tapered portion 111 and extending toward the axis-directional proximal end side of the tapered portion 111. Specifically, in the main body 113, for example, the distal end is continuous with the proximal end of the tapered portion 111, and the proximal end is connected to the base portion 31 described below. The main body 113 has a substantially constant outer diameter from the distal end to the proximal end. Incidentally, the main body 113 can be formed integrally with or separately from the tapered portion 111. In the present embodiment, the main body 113 and the tapered portion 111 are integrally formed by casting or the like.

Preferably, materials constituting the distal end portion 112, the tapered portion 111, and the main body 113 described above have antithrombogenicity, flexibility, and biocompatibility because they are inserted into a body cavity. For example, a resin material such as a polyamide resin, a polyolefin resin, a polyester resin, a polyurethane resin, a silicone resin, and a fluororesin; a metal material such as a stainless steel and a superelastic alloy (nickel-titanium alloy); or the like can be adopted.

Incidentally, the distal end portion 112, the tapered portion 111, and the main body 113 may have various coating films in the outer surface side part thereof (outer peripheral surface s11). Examples of the coating film include a protective film (plating film as a typical example) for the surfaces of the distal end portion 112, the tapered portion 111, and the main body 113, a base film for improving adhesiveness of the distal end portion 112, the tapered portion 111, and the main body 113 with the spirally-arranged protruding portion 21, and the like.

The spirally-arranged protruding portion 21 is formed on each of the outer peripheral surfaces of the tapered portion, and the distal end portion and/or the main body, and has gaps between adjacent sections along an axial direction of the shaft 11. In addition, the spirally-arranged protruding portion formed on the tapered portion has a height larger than heights of the spirally-arranged protruding portions formed on the distal end portion and/or the main body.

In the present embodiment, the spirally-arranged protruding portion 21 is composed of a spirally-arranged protruding portion 212 on the outer peripheral surface s11 of the distal end portion 112, a spirally-arranged protruding portion 211 on the outer peripheral surface s11 of the tapered portion 111, and a spirally-arranged protruding portion 213 on the outer peripheral surface s11 of the main body 113. The spirally-arranged protruding portion 21 projects from the outer peripheral surface s11 of the shaft 11 toward an outside in a radial direction (outermost surface and outermost portion of the dilator 1), and is arranged such that adjacent sections along the axial direction are distant. Thereby, the tapered portion 111 can be advanced toward the distal end side by a screw action of the spirally-arranged protruding portion 21 owing to rotation of the shaft 11. In addition, the spirally-arranged protruding portion 211 formed on the tapered portion 111 has a height t1 larger than heights t2 and t3 of the spirally-arranged protruding portions 212 and 213 formed on the distal end portion 112 and the main body 113. Thereby, the spirally-arranged protruding portion 211 is bitten into a living tissue to a greater degree compared to the spirally-arranged protruding portions 212 and 213, so that the screw action during expansion of a hole is enhanced.

Herein, the spirally-arranged protruding portion 21 can be formed as a single-thread or multi-thread projecting portion that is continuous or intermittent in the axial direction. In addition, the spirally-arranged protruding portion 21 can be formed either integrally with or separately from the shaft 11.

In the present embodiment, the spirally-arranged protruding portion 21 is formed as a continuous single-thread projecting portion, and the spirally-arranged protruding portion 21 is formed integrally with the distal end portion 112, the tapered portion 111, and the main body 113 by casting or the like.

Incidentally, it is preferable that the spirally-arranged protruding portion 21 does not constitute a blade (not have a shape that cuts the living tissue). That means, the spirally-arranged protruding portion 21 preferably has a sectional shape (transverse section orthogonal to the spiral direction of the spirally-arranged protruding portion 21) in which a radial outer end portion of the shaft 11 is not an acute-angled corner. Examples of such an end portion include an obtuse-angled corner, a part composed of a shape including a curved line (e.g. a curved line containing a part of a circle or an ellipse), and the like. Thereby, the dilator 1 can expand a hole formed on a wall of an organ or a constricted part (hole of an object) without damaging the living tissue on an inner face of the hole.

A material constituting the spirally-arranged protruding portion 21 is not particularly limited as long as the effect of the present invention is not impaired. For example, a metal material such as a stainless steel and a superelastic alloy (nickel-titanium alloy); a biocompatible resin material such as a polyamide resin and a fluororesin; or the like can be used.

Herein, as illustrated in FIG. 2, the outer periphery of the aforementioned shaft and/or spirally-arranged protruding portion may have a coating c. This coating c is formed for the purpose of improving e.g. slidability, preventing living tissue from biting into the shaft or spirally arranged protruding portion, or the like. In such a case, as a material for forming the coating c, for example, a biocompatible resin material such as a polyamide resin and a fluororesin, a hydrophilic coating material, or the like can be adopted. The coating c can have a thickness of e.g. 0.1 to 300 µm. Incidentally, when the shaft and/or the spirally-arranged protruding portion is coated, an outer peripheral surface s11m1 (outer surface) of the coating c is an outer peripheral surface of the shaft 11m1 (outer peripheral surfaces of a distal end portion 112m1, a tapered portion 111m1, and a main body 113m1), and an outer peripheral surface of a spirally-arranged protruding portion 21m1 (see a dilator 1m1).

The base portion 31 is a part where an operator pushes the dilator 1 into a body and/or rotates the dilator 1. This base portion 31 has the distal end connected to the proximal end of the main body 113 and has a through-hole 31h communicating with the through-hole 11h of the shaft 11. During the operation, a guide wire or the like is inserted through the through-hole 31h of the base portion 31.

Ordinarily, axial lengths of the respective parts of the dilator 1 are 1,600 to 2,500 mm in the whole shaft 11, 0 to 100 mm in the distal end portion 112, and 5 to 100 mm in the tapered portion 111. Ordinarily, outer diameters of the respective parts of the shaft 11 are 0.8 to 3.0 mm in the distal end portion 112 and the distal end of the tapered portion 111, and 1.4 to 5.0 mm in the proximal end of the tapered portion 112 and the main body 113. Ordinarily, an inner diameter of the through-hole 11h of the shaft 11 is 0.4 to 1.0 mm. Ordinarily, as for the heights of the spirally-arranged protruding portion 21, the height t2 in the distal end portion is 0.1 to 0.4 mm, the height t1 in the tapered portion is 0.125 to 0.5 mm, and the height t3 in the main body is 0.1 to 0.4 mm. Preferably, each ratio of the height t1 of the spirally-arranged protruding portion 211 to the height t2 or t3 of the spirally-arranged protruding portion 212 or 213 is larger than 1 and smaller than or equal to 5.

The axial lengths of the respective parts of the dilator 1 according to the present embodiment are 2,000 mm in the whole shaft 11, 10 mm in the distal end portion 112, and 30 mm in the tapered portion 111. The outer diameters of the respective parts of the shaft 11 are 1.84 mm in the distal end portion 112 and the distal end of the tapered portion 111, and 2.64 mm in the proximal end of the tapered portion 111 and the main body 113. The inner diameter of the through-hole 11h of the shaft 11 is 0.7 mm. As for the heights of the spirally-arranged protruding portion 21, the height t1 is 0.3 mm, the height t2 is 0.2 mm, the height t3 is 0.2 mm. As for the ratio of the height of the spirally-arranged protruding portion 211 to the height of the spirally-arranged protruding portion 212 or 213, t1/t2 is 1.5, or t1/t3 is 1.5 respectively.

Next, an example of how to use the dilator 1 will be explained.

First, the object is punctured with an introducer needle (not illustrated) to form a hole. Subsequently, a guide wire (not illustrated) is inserted into a through-hole of the introducer needle, and then the introducer needle is drawn out.

Next, the proximal end of the guide wire is inserted into the through-hole 11h of the dilator 1, the dilator 1 is pushed and advanced until the hole site (hereinafter, also referred to as "punctured portion") of the object, and the distal end portion 112 and the tapered portion 111 are inserted into the hole in this order. Subsequently, the tapered portion 111 is further pushed and advanced while rotating the shaft 11, to expand the hole. When the tapered portion 111 expands the hole, an indentation resistance particularly increases, but the dilator 1 is securely advanced to expand the hole by the enhanced screw action of the spirally-arranged protruding portion 211 on the outer periphery of the tapered portion 111.

As described above, since the dilator 1 has the aforementioned configuration, because of the larger height of the spirally-arranged protruding portion 211 on the tapered portion 111 that is particularly susceptible to a high resistance when expanding the hole of the object, this protruding portion 211 sufficiently engages with the object, and the dilator 1 can be advanced more strongly. Thus, for example, a hole, a constricted part, or the like formed on a wall of an organ or the like (hole of the object) can be easily and securely expanded. In addition, since the dilator 1 has the distal end portion 112, the hole can be smoothly expanded by the tapered portion 111 following the preceding distal end portion 112.

### [Second Embodiment]

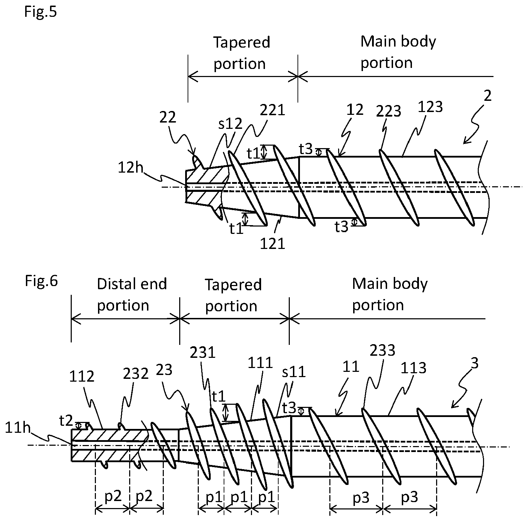

FIG. 5 is a partially cut-out schematic enlarged side view of a main part illustrating a second embodiment of the present invention. As illustrated in FIG. 5, a dilator 2 is roughly composed of a shaft 12, a spirally-arranged protruding portion 22, and the base portion 31 (not illustrated). The dilator 2 differs from the dilator according to the first embodiment in terms of the shaft 12 and the spirally-arranged protruding portion 22. Incidentally, since the configuration of the base portion 31 is the same as in the first embodiment, the same parts are given the same reference numerals as those in the first embodiment, and detailed explanation of the same parts is omitted. In addition, since the configurations excluding the shapes of the shaft 12 and the spirally-arranged protruding portion 22 are the same as in the first embodiment, explanation of their configurations in the first embodiment is applied to explanation thereof in the second embodiment.

The shaft 12 is a member having a hollow through-hole 12h. The shaft 12 according to the present embodiment does not have a distal end portion (e.g. see FIG. 1) but has a tapered portion 121 and a main body 123. Incidentally, since the configurations of the tapered portion 121 and the main body 123 are the same as in the first embodiment, explanation of their configurations in the first embodiment is applied to explanation thereof in the second embodiment.

The spirally-arranged protruding portion 22 is formed on each of the outer peripheral surfaces of the tapered portion and the main body, and has gaps between adjacent sections along the axial direction of the shaft 12. In addition, the spirally-arranged protruding portion 22 is formed such that a height t1 of a spirally-arranged protruding portion 221 formed on the tapered portion 121 is larger than a height t3 of a spirally-arranged protruding portion 223 formed on the main body 123. Thus, the spirally-arranged protruding portion 221 can be bitten into a living tissue to a greater degree compared to the spirally-arranged protruding portion 223, so that a screw action during expansion of a hole is enhanced.

As described above, since the dilator 2 has the aforementioned configuration, because of the larger height t1 of the spirally-arranged protruding portion 221 on the tapered portion 121 that is particularly susceptible to a high resistance when expanding the hole of the object, the spirally-arranged protruding portion 221 sufficiently engages with the object, and the dilator 2 can be advanced more strongly.

### [Third Embodiment]

FIG. 6 is a partially cut-out schematic enlarged side view of a main part illustrating the third embodiment of the present invention. As illustrated in FIG. 6, a dilator 3 is roughly composed of the shaft 11, a spirally-arranged protruding portion 23, and the base portion 31 (not illustrated). The dilator 3 differs from the dilator according to the first embodiment in terms of the spirally-arranged protruding portion 23. Incidentally, since the configurations of the shaft 11 and the base portion 31 are the same as in the first embodiment, the same parts are given the same reference numerals as those in the first embodiment, and detailed explanation of the same parts is omitted. In addition, since the configurations excluding the shape of the spirally-arranged protruding portion 23 are the same as in the first embodiment, explanation of their configurations in the first embodiment is applied to explanation thereof in the third embodiment.

The spirally-arranged protruding portion 23 is formed on each of the outer peripheral surfaces of the tapered portion, and the distal end portion and/or the main body, and has gaps between adjacent sections along an axial direction of the shaft 11. In addition, the spirally-arranged protruding portion formed on the tapered portion has a height larger than heights of the spirally-arranged protruding portions formed on the distal end portion and/or the main body.

In the present embodiment, the spirally-arranged protruding portion 23 is formed on the outer peripheral surfaces s11 of the distal end portion 112, the tapered portion 111 and the main body 113, and is formed such that a height t1 of a spirally-arranged protruding portion 231 formed on the tapered portion 111 is larger than heights t2 and t3 of spirally-arranged protruding portions 232 and 233 formed on the distal end portion 112 and the main body 113, and a pitch p1 of the spirally-arranged protruding portion 231 formed on the outer peripheral surface s11 of the tapered portion 111 is smaller than pitches p2 and p3 of the spirally-arranged protruding portions 232 and 233 formed on the outer peripheral surface s11 of the shaft 11 excluding the tapered portion 111 (on the outer peripheral surfaces s11 of the distal end portion 112 and the main body 113) as illustrated in FIG. 6.

Ordinarily, as for the pitches of the spirally-arranged protruding portion 23, the pitch p1 in the tapered portion is 0.2 to 4 mm, the pitch p2 in the distal end portion is 0.25 to 5 mm, and the pitch p3 in the main body is 0.25 to 5 mm. Preferably, each ratio of the pitch p1 of the protruding portion of the spiral 231 to the pitch p2 or p3 of the spirally-arranged protruding portion 232 or 233 is larger than 0.04 and less than 1.

In the present embodiment, as for the pitches of the spirally-arranged protruding portion 23, the pitch p1 is 1.5 mm, the pitch p2 is 2 mm, and the pitch p3 is 2 mm. As for the ratio of the pitch of the spirally-arranged protruding portion 231 to the pitch of the spirally-arranged protruding portion 232 or 233, p1/p2 is 0.75, or p1/p3 is 0.75.

As described above, the dilator 3 is formed such that the pitch p1 of the spirally-arranged protruding portion 231 is smaller than the pitches p2 and p3 of the spirally-arranged protruding portions 232 and 233. Thus, when the tapered portion 111 expands a hole of an object, because of the smaller pitch p1 of the spirally-arranged protruding portion 231 in the tapered portion 111, a stronger screw action can be provided, and the dilator 3 can be more reliably advanced.

### [Fourth Embodiment]

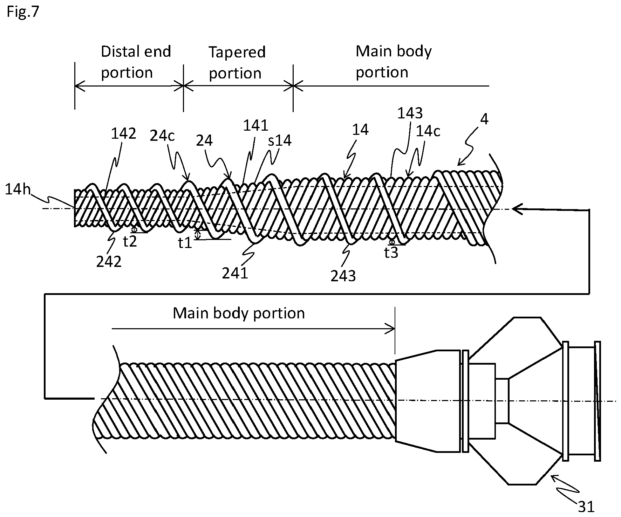

FIG. 7 is a schematic side view illustrating the whole of the fourth embodiment of the present invention. As illustrated in FIG. 7, a dilator 4 is roughly composed of a shaft 14, a spirally-arranged protruding portion 24, and the base portion 31. The dilator 4 differs from the dilator according to the first embodiment in terms of the shaft 14 and the spirally-arranged protruding portion 24. Incidentally, since the base portion 31 is the same as the base portion according to the first embodiment, the same parts are given the same reference numerals as those in the first embodiment, and detailed explanation of the same parts is omitted.

The shaft 14 is a hollow member having a through-hole 14h. The shaft 14 according to the present embodiment includes a tapered portion 141, a distal end portion 142, and a main body 143. The tapered portion 141 is a part having an outer diameter that is smaller at a distal end than at a proximal end. The distal end portion 142 is a part having a proximal end positioned on the distal end of the tapered portion 141 and extending toward an axis-directional distal end side of the tapered portion 141. The main body 143 is a part having a distal end positioned on the proximal end of the tapered portion 141 and extending toward the axis-directional proximal end side of the tapered portion 141.

The shaft 14 according to the present embodiment is composed of a first coil body 14c including a wire wound around an axis of the shaft 14. Specifically, as illustrated in FIG. 7, the shaft 14 is formed e.g. as the coil body 14c configured with one wire wound spirally such that adjacent wire parts are in close contact with each other in the axial direction. The first coil body 14c is formed in the aforementioned distal end portion 142, tapered portion 141, and main body 143 according to a shape of an outer peripheral face s14 of the first coil body 14c. Incidentally, in FIG. 7, common internal tangents of the first coil body 14c are illustrated with dashed lines, and the through-hole 14h is formed in a region surrounded by the common internal tangents.

The spirally-arranged protruding portion 24 is formed on each of the outer peripheral surfaces of the tapered portion, and the distal end portion and/or the main body, and has gaps between adjacent sections along an axial direction of the shaft 14. The spirally-arranged protruding portion formed on the tapered portion has a height larger than heights of the spirally-arranged protruding portions formed on the distal end portion and/or the main body.

The spirally-arranged protruding portion 24 according to the present embodiment is composed of a second coil body 24c including a wire wound around on the outer peripheral surface s14 (outer peripheral surfaces s14 of the distal end portion 142, the tapered portion 141 and the main body 143) of the shaft 14. The spirally-arranged protruding portion 24 is formed on each of the outer peripheral surfaces s14 of the tapered portion 141, the distal end portion 142, and the main body 143. A spirally-arranged protruding portion 241 formed on the tapered portion 141 has a height t1 larger than heights t2 and t3 of spirally-arranged protruding portions 242 and 243 formed on the distal end portion 142 and the main body 143.

The spirally-arranged protruding portion 24 can be composed of one wire or a plurality of wires, in which the adjacent wire parts are distant from each other. When the second coil body 24c is composed of one wire, the second coil body 24c can be configured e.g. such that a diameter of the wire of the second coil body 24c wound around on the outer peripheral surface s14 of the tapered portion 141 is larger than a diameter of the wire of the second coil body 24c wound around on the outer peripheral surface s14 of the shaft 14 excluding the tapered portion 141. Thereby the height t1 of the spirally-arranged protruding portion 241 with respect to the heights t2 and t3 of the spirally-arranged protruding portions 242 and 243 can be easily adjusted by changing diameters of one wire for the respective portions. For the purpose of performing such an adjustment, it is possible to use a wire prepared e.g. by polishing a part (part constituting the protruding portions 242 and 243) of a wire rod used as a wire material for the second coil body 24c or by coating another part (part constituting the protruding portion 241). The material used for the coating can be exemplified by the same materials as cited for the materials of the coating c illustrated in FIG. 2. On the other hand, when the second coil body 24c is composed of a plurality of wires, for example, the spirally-arranged protruding portion 24 can be formed using wires having different diameters, and for example, the second coil body 24c can be easily obtained by combining ready-made wire rods.

The diameter of the wire constituting the first coil body 14c is ordinarily 0.1 to 0.5 mm. The diameter of the wire constituting the second coil body 24c, and a ratio of the height t1 of the spirally-arranged protruding portion 241 to the height t2 or t3 of the spirally-arranged protruding portion 242 or 243 can be set to the same values of the height of the spirally-arranged protruding portion 21, and the ratio of the height t1 of the spirally-arranged protruding portion 211 to the height t2 or t3 of the spirally-arranged protruding portion 212 or 213 respectively explained in the first embodiment. In the present embodiment, the wire of the first coil body 14c has a diameter of 0.21 mm. The second coil body 24c is composed of one wire, and the diameters of the wire are 0.2 mm in the distal end portion 142, 0.3 mm in the tapered portion 141, and 0.2 mm in the main body 143.

As a method for joining the first coil body 14c and the second coil body 24c, for example, a method of brazing the end portions of them, a welding method, a fixing method using an adhesive, an adhering method by welding with a coating film, or the like can be adopted.

Incidentally, it is preferable that the winding direction of the wire in the first coil body 14c and the winding direction of the wire in the second coil body 24c are opposite to each other as in the present embodiment. That means, either one of the first coil body 14c and the second coil body 24c is S-twisted and the other is Z-twisted. Thereby, when rotating the shaft 14, directions of the axial forces applied to the wires of the first coil body 14c and the second coil body 24c can be made opposite to each other, and decrease in torquability and pushability due to distancing between the adjacent wires in the first coil body 14c can be prevented.

The material for the wire constituting the aforementioned first coil body 14c can be exemplified by the same materials as cited for the materials constituting the distal end portion 112, the tapered portion 111, and the main body 113 in the first embodiment. The material for the wire constituting the aforementioned second coil body 24c can be exemplified by the same materials as cited for the materials constituting the spirally-arranged protruding portion 21 in the first embodiment.

As described above, in the dilator 4, the shaft 14 and the spirally-arranged protruding portion 24 are composed of the first coil body 14c and the second coil body 24c respectively, therefore flexibility and torquability can be improved in each of the shaft 14 and the spirally-arranged protruding portion 24, and furthermore flexibility and torquability can be further improved by their synergistic action.

### [Fifth Embodiment]

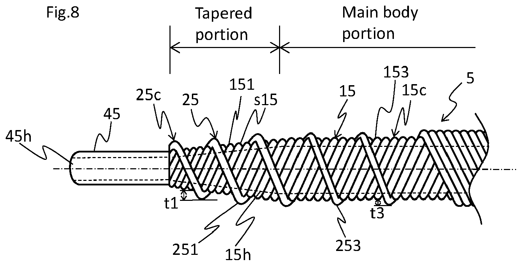

FIG. 8 is a schematic enlarged side view of a main part illustrating the fifth embodiment of the present invention. As illustrated in FIG. 8, a dilator 5 is roughly composed of a shaft 15, a spirally-arranged protruding portion 25, a distal tip 45, and the base portion 31 (not illustrated). The dilator 5 differs from the dilator according to the third embodiment in terms of the shaft 15, the spirally-arranged protruding portion 25, and the distal tip 45. Incidentally, since the base portion 31 is the same as in the first embodiment, detailed explanation of the base portion 31 is omitted.

The shaft 15 is a member having a hollow through-hole 15h. The shaft 15 according to the present embodiment has a tapered portion 151 and a main body 153. The spirally-arranged protruding portion 25 is formed on each outer peripheral surface s15 of the tapered portion 151 and the main body 153, and has gaps between adjacent sections along an axial direction of the shaft 15. In addition, a spirally-arranged protruding portion 251 formed on the tapered portion 151 has a height t1 larger than a height t3 of a spirally-arranged protruding portion 253 formed on the main body 153. Incidentally, since configurations of the tapered portion 151 and the main body 153 in the shaft 15 and a configuration of the spirally-arranged protruding portion 25 are the same as in the fourth embodiment, explanation of their configurations in the fourth embodiment is applied to explanation thereof in the fifth embodiment..

The distal tip 45 is a part having a proximal end positioned on the distal end of the shaft 15 and extending toward the axis-directional distal end side of the tapered portion 151. The distal tip 45 has a surface flatter than an outer peripheral surface s15 (uneven surface due to the wire) of a first coil body 15c, and includes an substantially hollow cylindrical through-hole 45h communicating with the through-hole 15h of the shaft 15. Incidentally, in the present embodiment, an inner diameter of the through-hole 45h of the distal tip 45 is 0.7 mm, and inner diameters of the through-hole 15h of the shaft 15 are 0.7 mm on the distal end, and 1.5 mm on the proximal end.

The distal tip 45 can be formed e.g. by flattening the surfaces of the distal end portions of the first coil body 15c and a second coil body 25c using a solder material such as a silver-tin solder and a gold-tin solder, or the like

As described above, in the dilator 5, the shaft 15 and the spirally-arranged protruding portion 25 are composed of the first coil body 15c and the second coil body 25c respectively, and therefore flexibilty and torquability of the shaft 15 and the spirally-arranged protruding portion 25 can be improved. In addition, since the dilator 5 includes a distal tip 45, insertability of the shaft 15 into a hole bored by an introducer needle can be improved, and the procedure can be smoothly advanced.

### [Sixth Embodiment]

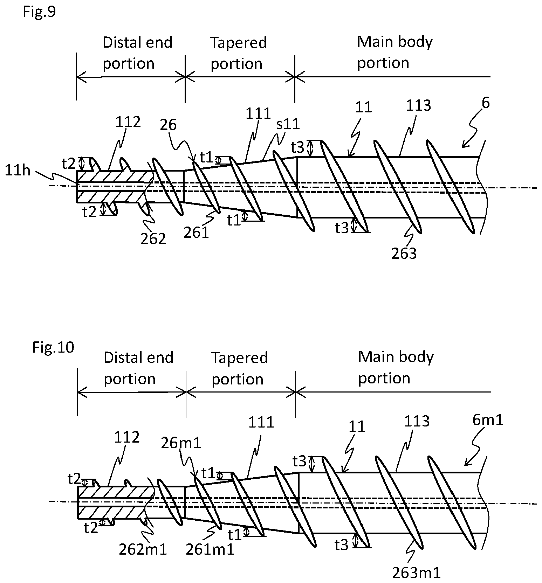

FIG. 9 is a partially cut-out schematic enlarged side view of a main part illustrating the sixth embodiment of the present invention. As illustrated in FIG. 9, a dilator 6 is roughly composed of the shaft 11, a spirally-arranged protruding portion 26, and the base portion 31 (not illustrated). The dilator 6 differs from the dilator according to the first embodiment in terms of the spirally-arranged protruding portion 26. Incidentally, since the configurations of the shaft 11 and the base portion 31 are the same as in the first embodiment, the same parts are given the same reference numerals as those in the first embodiment, and detailed explanation of the same parts is omitted.

The spirally-arranged protruding portion 26 is formed on each of the outer peripheral surfaces of the tapered portion, and the distal end portion and/or the main body, and has gaps between adjacent sections along an axial direction of the shaft 11. The spirally-arranged protruding portion 26 is formed such that a height of the spirally-arranged protruding portion formed on the tapered portion is smaller than heights of the spirally-arranged protruding portions formed on the distal end portion and/or the main body.

In the present embodiment, the spirally-arranged protruding portion 26 is formed on each of the outer peripheral surfaces s11 of the distal end portion 112, the tapered portion 111, and the main body 113 such that a height t1 of a spirally-arranged protruding portion 261 formed on the tapered portion 111 is smaller than heights t2 and t3 of spirally-arranged protruding portions 262 and 263 formed on the distal end portion 112 and the main body 113.

Ordinarily, as for the heights of the spirally-arranged protruding portion 26, the height t2 in the distal end portion is 0.125 to 0.5 mm, the height t1 in the tapered portion is 0.1 to 0.4 mm, and the height t3 in the main body is 0.125 to 0.5 mm. Preferably, each ratio of the height t1 of the spirally-arranged protruding portion 261 to the height t2 or t3 of the spirally-arranged protruding portion 262 or 263 is larger than 0.2 and less than 1.

As for the heights of the spirally-arranged protruding portion 26 according to the present embodiment, the height t1 is 0.2 mm, the height t2 is 0.3 mm, and the height t3 is 0.3 mm. As for the ratio of the height t1 of the spirally-arranged protruding portion 261 to the height t2 or t3 of the spirally-arranged protruding portion 262 or 263, t1/t2 is 0.7, or t1/t3 is 0.7.

Next, an example of how to use the dilator 6 will be explained. Incidentally, since formation of a hole by puncturing an object part, and insertion of the dilator 6 into the punctured part using a guide wire are the same as in the first embodiment, explanation thereof in the first embodiment is applied to explanation thereof in the sixth embodiment.

After inserting the dilator 6 until the punctured part, the distal end portion 112 and the tapered portion 111 are inserted into the hole in this order. Subsequently, the tapered portion 111 is further pushed and advanced while rotating the shaft 11, to expand the hole. When this tapered portion 111 expands the hole, use of the dilator 6 makes it possible to push the shaft 11 also in a linear manner while utilizing a screw action of the spirally-arranged protruding portion 26, so that the dilator 6 smoothly advances to expand the hole.

As described above, since the dilator 6 has the aforementioned configuration, because of the smaller height t1 of the spirally-arranged protruding portion 261 on the tapered portion 111 that is particularly susceptible to a high resistance when expanding the hole of the object, an indentation resistance can be decreased by alleviating inroad of the spirally-arranged protruding portion into a living tissue, and the hole of the object can be effectively expanded by combining the screw action and the pushing. In addition, since the dilator 6 has the distal end portion 112, the hole can be smoothly expanded by the tapered portion 111 following the preceding distal end portion 112.

### [Seventh Embodiment]

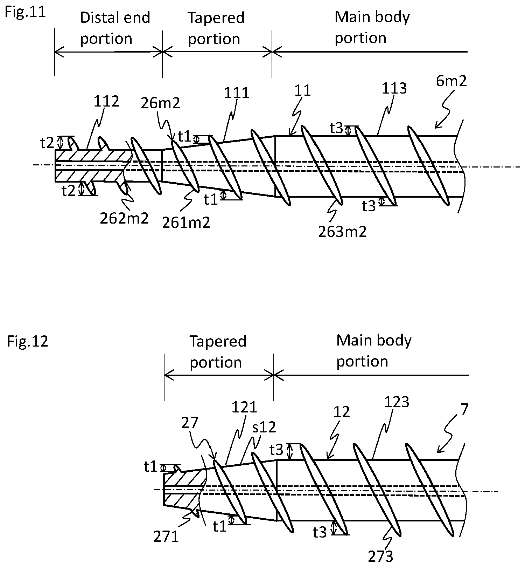

FIG. 12 is a partially cut-out schematic enlarged side view of a main part illustrating the seventh embodiment of the present invention. As illustrated in FIG. 12, a dilator 7 is roughly composed of a shaft 12, a spirally-arranged protruding portion 27, and the base portion 31 (not illustrated). The dilator 7 differs from the dilator according to the second embodiment in terms of the spirally-arranged protruding portion 27. Incidentally, since the configurations of the shaft 12 and the base portion 31 are the same as in the second embodiment, the same parts are given the same reference numerals as those in the second embodiment, and detailed explanation of the same parts is omitted. In addition, since how to use the dilator 7 is the same as in the sixth embodiment, explanation thereof in the sixth embodiment is applied to explanation thereof in the seventh embodiment.

The spirally-arranged protruding portion 27 is formed on each of outer peripheral surfaces s12 of the tapered portion 121 and the main body 123, and has gaps between adjacent sections along an axial direction of the shaft 12. The spirally-arranged protruding portion 27 is formed such that a height t1 of a spirally-arranged protruding portion 271 formed on the tapered portion 121 is smaller than a height t3 of a spirally-arranged protruding portion 273 formed on the main body 123. Incidentally, since the configurations of the spirally-arranged protruding portions 271 and 273 are the same as the configurations of the spirally-arranged protruding portions 261 and 263 respectively in the sixth embodiment, explanation of the same configurations is omitted in the seventh embodiment.

As described above, since the dilator 7 has the aforementioned configuration, an indentation resistance can be decreased by alleviating inroad of the spirally-arranged protruding portion into a living tissue, and a hole of an object can be effectively expanded by combining a screw action and pushing.

Note that the present invention are not limited to the configurations of the aforementioned embodiments, but is indicated by claims, the present invention are intended to include all modifications within the meaning and scope equivalent to those in claims.

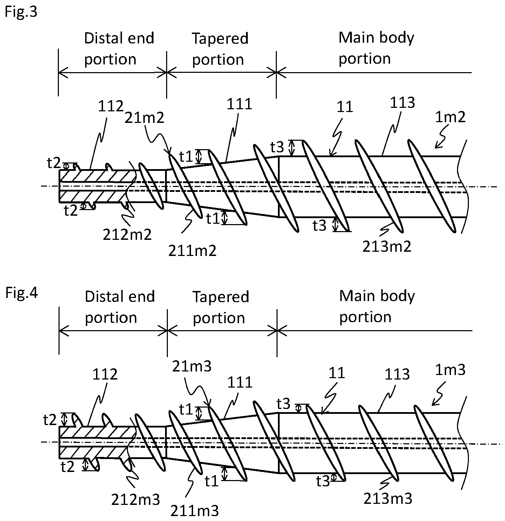

For example, in the aforementioned first embodiment, the dilator 1 in which the height t1 of the spirally-arranged protruding portion 211 is larger than the heights t2 and t3 of the spirally-arranged protruding portions 212 and 213 (see FIG. 1) has been explained. However, the dilator may be a dilator in which the height of the spirally-arranged protruding portion formed on the tapered portion is larger than the height of the spirally-arranged protruding portion formed on the distal end portion regardless of the height of the spirally-arranged protruding portion formed on the main body (exemplified by a dilator 1m2 with height t2 of spirally-arranged protruding portion 212m2<height t1 of spirally-arranged protruding portion 211m2=height t3 of spirally-arranged protruding portion 213m2 in FIG. 3), or may be a dilator in which the height of the spirally-arranged protruding portion formed on the tapered portion is larger than the height of the spirally-arranged protruding portion formed on the main body regardless of the height of the spirally-arranged protruding portion formed on the distal end portion (exemplified by a dilator 1m3 with height t2 of spirally-arranged protruding portion 212m3=height t1 of spirally-arranged protruding portion 211m3>height t3 of spirally-arranged protruding portion 213m3 in FIG. 4).

Additionally, in the aforementioned sixth embodiment, the dilator 6 in which the height t1 of the spirally-arranged protruding portion 261 is smaller than the heights t2 and t3 of the spirally-arranged protruding portions 262 and 263 (see FIG. 9) has been explained. However, the dilator may be a dilator in which the height of the spirally-arranged protruding portion formed on the tapered portion is smaller than the height of the spirally-arranged protruding portion formed on the main body regardless of the height of the spirally-arranged protruding portion formed on the distal end portion (exemplified by a dilator 6m1 with height t2 of spirally-arranged protruding portion 262m1=height t1 of spirally-arranged protruding portion 261<height t3 of spirally-arranged protruding portion 263m1 in FIG. 10), or may be a dilator in which the height of the spirally-arranged protruding portion formed on the tapered portion is smaller than the height of the spirally-arranged protruding portion formed on the distal end portion regardless of the height of the spirally-arranged protruding portion formed on the main body (exemplified by a dilator 6m2 with height t2 of spirally-arranged protruding portion 262m2>height t1 of spirally-arranged protruding portion 261m2=height t3 of spirally-arranged protruding portion 263m2 in FIG. 11).

Furthermore, in the aforementioned first embodiment, the dilator 1m1 having the coating c in the outer peripheral side part of the shaft 11 and the spirally-arranged protruding portion 21 has been explained, and in the fourth embodiment, the dilator 5 having the distal tip 45 has been explained. However, each of the coating c and the distal tip 45 may be applied to the dilators according to any embodiments other than the embodiments described above.

### REFERENCE SIGNS LIST

- 1 to 7: Dilator
- 11, 12, 14,: 15 Shaft
- 111, 121, 141, 151: Tapered portion
- 112, 142: Distal end portion
- 113, 123, 143, 153: Main body
- 14c, 15c: First coil body
- 21 to 27: Spirally-arranged protruding portion
- 24c, 25c: Second coil body

## Claims

1. A dilator comprising a hollow shaft and a spirally-arranged protruding portion, wherein
the shaft comprises either
a tapered portion having an outer diameter that is smaller at a distal end of the tapered portion than at a proximal end of the tapered portion,
a distal end portion having a proximal end positioned on a distal end of the tapered portion and extending toward an axis-directional distal end side of the tapered portion, and
a main body having a distal end positioned on a proximal end of the tapered portion and extending toward an axis-directional proximal end side of the tapered portion, or the tapered portion and the main body without the distal end portion, and
in a case where the shaft does not have the distal end portion, the spirally-arranged protruding portion is formed on each of outer peripheral surfaces of the tapered portion and the main body and has gaps between adjacent sections of the spirally-arranged protruding portion along an axial direction of the shaft, and
the spirally-arranged protruding portion formed on the tapered portion has a height larger than a height of the spirally-arranged protruding portion formed on the main body, and
in a case where the shaft has the distal end portion, the spirally-arranged protruding portion is formed on each of outer peripheral surfaces of the tapered portion, and the distal end portion and/or the main body, and has gaps between adjacent sections of the spirally-arranged protruding portion along the axial direction of the shaft, and
the spirally-arranged protruding portion formed on the tapered portion has a height larger than heights of the spirally-arranged protruding portions formed on the distal end portion and/or the main body.

2. The dilator according to claim 1, wherein the shaft includes a first coil body comprising a wire wound around an axis of the shaft.

3. The dilator according to claim 1 or 2, wherein the spirally-arranged protruding portion includes a second coil body comprising a wire wound around on the outer peripheral surface of the shaft.

4. The dilator according to 1, wherein
the shaft is composed of a first coil body comprising a wire wound around an axis of the shaft, and the spirally-arranged protruding portion is composed of a second coil body comprising a wire wound around on the outer peripheral surface of the shaft, and
a winding direction of the wire on the first coil body and a winding direction of the wire on the second coil body are opposite to each other.

5. The dilator according to claim 3 or 4, wherein
the wire of the second coil body wound around the outer peripheral surface of the tapered portion has a diameter larger than a diameter of the wire of the second coil body wound around the outer peripheral surface of the shaft excluding the tapered portion.

6. The dilator according to any one of claims 1 to 5, wherein a pitch of the spirally-arranged protruding portion formed on the outer peripheral surface of the tapered portion is smaller than a pitch of the spirally-arranged protruding portion formed on the outer peripheral surface of the shaft excluding the tapered portion.

7. A dilator comprising a hollow shaft and a spirally-arranged protruding portion, wherein
the shaft comprises either
a tapered portion having an outer diameter that is smaller at a distal end of the tapered portion than at a proximal end of the tapered portion,
a distal end portion having a proximal end positioned on a distal end of the tapered portion and extending toward an axis-directional distal end side of the tapered portion, and
a main body having a distal end positioned on a proximal end of the tapered portion and extending toward an axis-directional proximal end side of the tapered portion, or
the tapered portion and the main body without the distal end portion, and
in a case where the shaft does not have the distal end portion, the spirally-arranged protruding portion is formed on each of outer peripheral surfaces of the tapered portion and the main body and has gaps between adjacent sections of the spirally-arranged protruding portion along an axial direction of the shaft, and
the spirally-arranged protruding portion formed on the tapered portion has a height smaller than a height of the spirally-arranged protruding portion formed on the main body, and
in a case where the shaft has the distal end portion, the spirally-arranged protruding portion is formed on each of outer peripheral surfaces of the tapered portion, and the distal end portion and/or the main body, and has gaps between adjacent sections of the spirally-arranged protruding portion along the axial direction of the shaft, and
the spirally-arranged protruding portion formed on the tapered portion has a height smaller than heights of the spirally-arranged protruding portions formed on the distal end portion and/or the main body.
